# EUROPEAN PATENT SPECIFICATION

(11) **EP 1 364 627 B1**
(45) Date of publication and mention of the grant of the patent: **12.09.2007**
(21) Application number: 03019053.2
(22) Date of filing: 17.02.1995
(51) Int. Cl.: A61F 2/06

(54) **Method for producing a bioremodelable collagen graft prosthesis**
Verfahren zur Herstellung einer Bio-umbaubare Transplantatprothese aus Kollagen
Procédé pour produire une greffe prothétique bioremodelable en collagène

(30) Priority: 18.02.1994 US 198062
(43) Date of publication of application: 26.11.2003
(62) Divisional of application: 95911032.1
(73) Proprietor: ORGANOGENESIS INC., Canton, MA 02021 (US)
(72) Inventor: Termin. Paul, L., St. Paul, MN 55105 (US); Carr, Robert, M, Jr., Paradise Valley, AZ 85253 (US); Condon, Kimberlie, D., Brant Rock, MA 02020 (US)
(74) Representative: Bassil, Nicholas Charles

(56) References cited:
- EP-A- 0 397 500
- EP-A- 0 493 788
- WO-A-89/10100
- GB-A- 2 153 235
- US-A- 4 539 716
- US-A- 4 902 289
- US-A- 4 902 508
- US-A- 5 024 671
- US-A- 5 026 381
- US-A- 5 106 949
- US-A- 5 256 418
- US-A- 5 263 983

## Description

### Field of the Invention

This invention is in the field of implantable biological prostheses. The present invention relates to a method for preparing resilient, biocompatible two- or three-layered tissue prosthesis which can be engineered in flat sheets or in tubes with various luminal diameters and thicknesses. At least one layer is composed of collagen or a collagenous material. The prostheses prepared by the method of the present invention is gradually degraded and bioremodeled by the host's cells which replace the implanted prosthesis and assume its shape.

### BACKGROUND OF THE INVENTION

Each year approximately 300,000 coronary bypass procedures are performed in the United States. The typical treatment for small diameter artery replacement has been for surgeons to use the patient's own vessels, usually the saphenous vein from the leg. However, in many cases, the use of the patient's own vessels is not practical because the veins are either damaged, diseased or are not available. In these cases, synthetic materials are used, but with unsatisfactory long-term results. It is still a continuing goal of researchers to develop prostheses which can successfully be used to replace or repair mammalian tissue, particularly blood vessels.

US-A- 4902289 describes a multilayer blood vessel prosthesis which consists of a relatively smooth and non-porous inner layer and a highly porous outer layer. US-A-4902508 describes a tissue graft composition comprising the tunica submucosa, the muscular is mucosa and the stratum compactum of the tunica mucosa derived from intestinal tissue. EP-A-0493788 describes a three layer prosthesis in which the middle layer of non-resorbable material preferably incorporates synthetic fibres. US-A-4787900 describes a process for forming a multilayer blood vessel prosthesis.

### SUMMARY OF THE INVENTION

This invention is directed to a method for preparing a bioremodelable prosthesis having at least two layers, comprising:
a) forming a first structural layer that is pliable, semi-permeable, and suturable; and
b) forming a second layer to act as a smooth flow surface comprising deposition of acid extracted fibrillar or non-fibrillar collagen onto a surface of said structural layer of step a).

Preferred features of the present invention are defined in dependent claims 2 to 26.

### BRIEF DESCRIPTION OF THE FIGURES

Figure 1A, 1B and 1C are schematic cross-sectional views of the preferred prosthesis prepared by the method in accordance with the present invention.

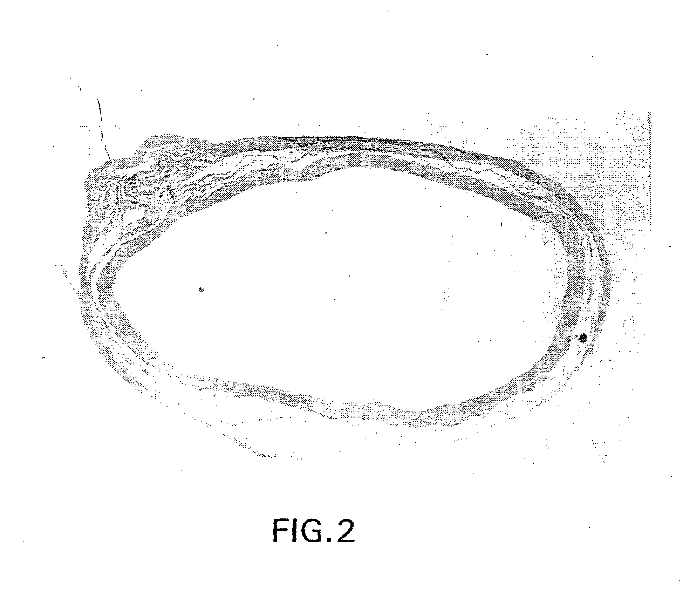

Figure 2 is a Masson's trichrome stain (10x)of a three-layer prosthesis prepared by the method of this invention prior to implantation.

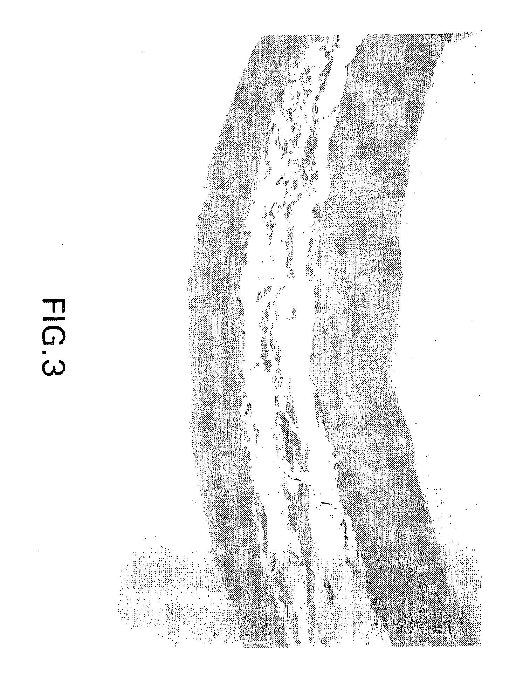

Figure 3 is a Masson's trichrome stain (25x) of a three-layer prosthesis prepared by the method of this invention prior to implantation.
Figure 4 is a Masson's trichrome stain (10x) of the proximal anastomosis of a three-layer prosthesis prepared by the method of this invention implanted as a canine femoral interposition prosthesis (256 days).
Figure 5 is a Masson's trichrome stain (10x) of the proximal anastomosis of an e-PTFE graft implanted as a canine femoral interposition prosthesis (256 days).
Figure 6 is a Masson's trichrome stain (25x) of the proximal anastomosis of a three-layer prosthesis prepared by the method of this invention implanted as a canine femoral interposition prosthesis (256 days).
Figure 7 is a Masson's trichrome stain (25x) of the proximal anastomosis of an e-PTFE graft implanted as a canine femoral interposition prosthesis (256 days).
Figure 8 is a Verhoeffs elastic stain (10x) of the proximal anastomosis of a three-layer prosthesis prepared by the method of this invention implanted as a canine femoral interposition prosthesis (256 days).
Figure 9 is a Verhoeffs elastic stain (10x) of the proximal anastomosis of an e-PTFE graft implanted as a canine femoral interposition prosthesis (256 days).

### DETAILED DESCRIPTION OF THE INVENTION

This invention is directed to a method for preparing bioremodelable prostheses, which, when implanted into a mammalian host, serve as a functioning replacement for a body part, or tissue structure, and will undergo controlled biodegradation occurring concomitantly with bioremodeling by the host's cells. The prosthesis prepared by the method of this invention, in its various embodiments, thus has dual properties: First, it functions as a substitute body part and second, while still functioning as a substitute body party, it functions as a bioremodeling template for the ingrowth of host cells.

When the prosthesis functions as a substitute body part, it is preferably used as a vascular graft. The vascular graft prosthesis may be tubular or flat. Tubular grafts will be used as a conduit to bypass or replace arteries or veins. When formed into flat sheets, the prosthesis can be used as a vascular or intra-cardiac patch. In addition, the prosthesis can be implanted to replace diseased or damaged organs, including the esophagus, intestine, bowel, urethra, and fallopian tubes. These organs all have a basic tubular shape with an outer surface and a luminal surface. Further, the prosthesis can be used as a conduit for nerve regrowth and regeneration.

The prosthesis prepared by the method of this invention has increased resiliency or "spring-open" or "spring-back" properties. Spring back properties are important for applications such as a vascular tubes or patches.

The second function of the prosthesis is that of a template for bioremodeling. "Bioremodeling" is used herein to mean the production of structural collagen, vascularization, and epithelialization by the ingrowth of host cells at a rate faster than the loss of biomechanical strength of the implanted prosthesis due to biodegradation by host enzymes. The prosthesis retains the distinct characteristics of the originally implanted prosthesis while it is remodeled by the body into all, or substantially all, "self" and as such is functional as a functioning tissue structure.

The prosthesis is made of at least two layers: (a) at least one layer is composed of collagen or a collagenous material that has a smooth, uniform diameter geometry and is nonthrombogenic and (2) at least one layer which provides structural stability and biomechanical properties. The mechanical integrity means that the prosthesis is non-dilating and non-aneurysmal during bioremodeling, and additionally is pliable and suturable. The term "pliable" means good handling properties. The term "suturable" means that the mechanical properties of the layer include suture retention which permits needles and suture materials to pass through the prosthesis material at the time of suturing of the prosthesis to sections of natural vessel, a process known as anastomosis. During suturing, such vascular (blood vessel) grafts must not tear as a result of the tensile forces applied to them by the suture, nor should they tear when the suture is knotted. Suturability of vascular grafts, i.e., the ability of grafts to resist tearing while being sutured, is related to the intrinsic mechanical strength of the prosthesis material, the thickness of the graft, the tension applied to the suture, and the rate at which the knot is pulled closed.

The prosthesis prepared by the method of this invention is particularly directed to use as a bypass or replacement of small diameter blood vessels in the host patient. As used herein, and as is understood by those of skill in the art, a small diameter tube is less than 6 mm, typically around 3 to 4 mm. A medium diameter tube is between 6 to 12 mm. A large diameter tube is greater than 12 mm. As an example, the various vascular diameter sizes in adult humans are as follows: the diameter of aortic vessels is from about 12 to 22 mm; the diameter of the iliac vein is from 8 to 12 mm; the diameter of the superficial femoral vein is 6 mm. Above the knee, the femoral is 6 mm; across the knee, the femoral is 4 to 6 mm.

The combination of the two layers of the prosthesis prepared by the method of this invention when used as a tubular vascular graft work advantageously by combining a smooth thrombosis resistant flow surface on the inner (luminal) collagenous layer with the structural layer which, in addition to its other properties, aids in preventing luminal creep, that is maintaining the nominal diameter. Dilatation (or aneurysmal) failure occurs when the pulsatile pressure and forces exceed the ability of the graft to resist an increase in diameter. Dilatation or aneurysm formation is an increase in diameter beyond nominal. This occurs in both prosthesis as well as in atherosclerotic arteries. As used herein, the term "non-dilatating" means that the biomechanical properties of the prosthesis impart durability so that the diameter of the prosthesis is not stretched, distended, or expanded beyond normal limits after implantation. As is described below, total dilatation of the implanted prosthesis prepared by the method of this invention is within acceptable limits. The prosthesis prepared by the method of this invention acquires a resistance to dilatation as a function of post-implantation cellular bioremodeling by replacement of structural collagen by host cells at a faster rate than the loss of mechanical strength of the implanted materials due from biodegradation and remodeling.

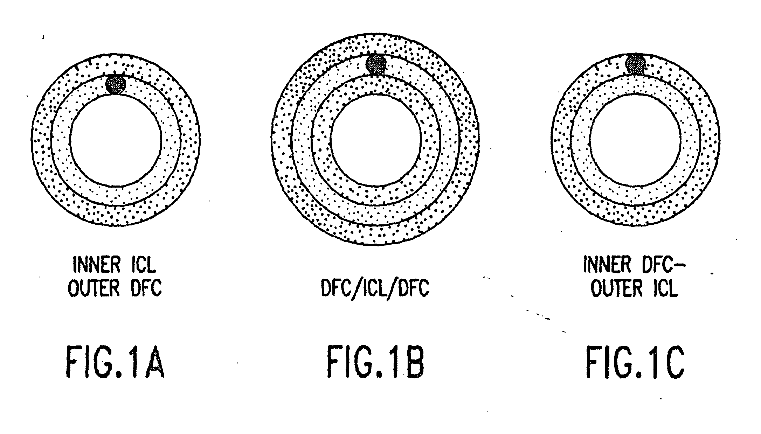

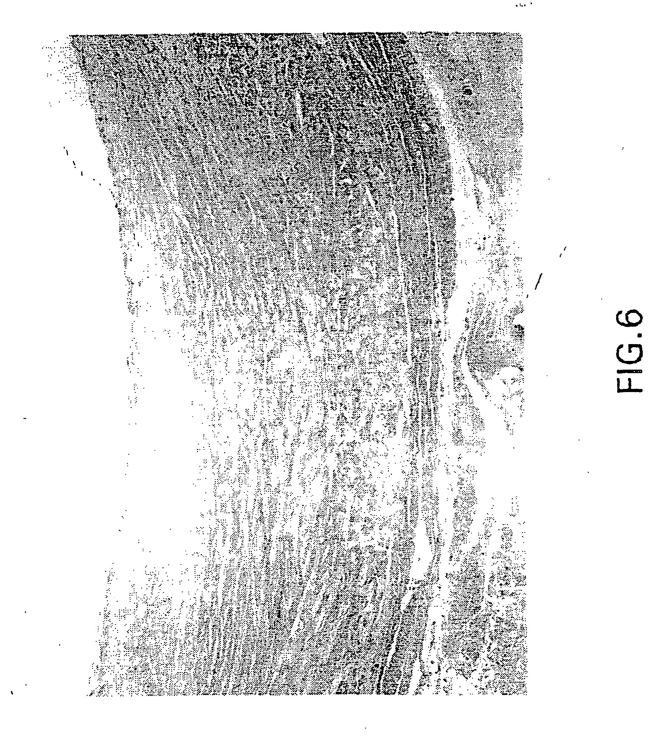

Various tubular configurations are embodied by this prosthesis as shown in Figures 1A, 1B, and 1C. Figure 1A shows a two layer prosthesis with an outer collagenous layer and an inner structural layer. Figure 1B shows a prosthesis with three layers: inner and an outer collagenous layers and a middle structural layer. Figure 1C shows a two layer prosthesis with an inner collagenous layer and an outer structural layer.

Each of these various embodiments has applicability for particular graft replacements. The two layer prosthesis shown by Figure 1A, with an outer collagenous layer and the inner structural layer, is useful as a replacement for vessels or hollow organs which can tolerate a less smooth inner or luminal surface, such as the esophagus, intestine, bowel, urethra, or fallopian tubes. The outer collagenous layer adds strength to the graft and allows the host's cells to attach to it, permitting ingrowth into the graft. In contrast, the prosthesis as shown in Figure 1B and in Figure 1C with an inner, smooth collagenous layer are useful as blood vessel replacements. The inner collagenous layer functions as a smooth flow surface.

The structural layer may be made from bioremodelable collagen or collagenous materials; or biodegradable polymeric materials, such as polylactic or polyglycolic acid, or combinations thereof; or biostable polymers, such as polytetrafluoroethylene (PTFE), polyethylene, or combinations thereof. In the preferred embodiment, collagenous material from collagenous parts of tissue from the mammalian body is used to make this layer. Such tissue includes but is not limited to intestine, fascia lata, or dura mater. The most preferred material for use as a structural layer is the tunica submucosa layer of the small intestine, termed herein the "intestinal collagen layer." As used herein, the structural layer will typically have a thickness of between about 50 microns to about 150 microns, more preferably between about 75 microns to about 125 microns. These dimensions are for a intestinal collagen layer after mechanical cleaning, but before tubulation by heat welding and crosslinking, as described below; both mechanical cleaning and heat welding significantly reduce the "apparent" thickness of the intestinal collagen layer.

When collagenous material of tissue origin is used to form the structural layer, it may be crosslinked to provide strength to the structure. Crosslinking collagenous material also provides some stiffness to the material to improve handling properties. Additionally, crosslinking collagenous material on a mandrel yields a tube of a more uniform diameter than if the material had not been crosslinked. This minimizes the risk of thrombosis which can be enhanced when there is discontinuity in the geometry of the vessel. Crosslinking agents should be selected so as to produce a biocompatible material capable of being bioremodeled by host cells. Various types of crosslinking agents are known and can be used; this is discussed below with the preferred embodiment. A preferred crosslinking agent is 1-ethyl-3-(3-dimethylaminopropyl) carbodiimide hydrochloride (EDC). There are certain crosslinking agents that cannot be used on the prosthesis prepared by the method of this invention since they will produce a crosslinked material that will not undergo remodeling by host cells. Glutaraldehyde, for example, is not useful for crosslinking with this invention as the residual of the glutaraldehyde monomer and lower molecular polymers are cytotoxic. Therefore, it would prevent cell ingrowth and bioremodeling.

The structural layer, at least when made with bioremodelable collagen or collagenous materials, such as the intestinal collagen layer, will be "semi-permeable", that is, permitting the ingrowth of host cells for remodeling or for deposition of the collagenous layer, as described below. Crosslinking ICL renders the material relatively less permeable as measured by water porosity testing.

The other layer of the prosthesis is the collagenous layer, the function of which is to act as a smooth flow surface for whatever its ultimate application. When used as the inner, luminal layer of the prosthesis, its function is to provide a smooth contacting surface, particularly a blood contact flow surface.

This smooth collagenous layer is made from acid-extracted fibrillar or non-fibrillar collagen, which is predominantly type I collagen, but may also include type 3 or 4 collagen, or both. The collagen used may be derived from any number of mammalian sources, typically bovine, porcine, or ovine skin and tendons. The collagen preferably has been processed by acid extraction to result in a fibril dispersion or gel of high purity. Collagen may be acid-extracted from the collagen source using a weak acid, such as acetic, citric, or formic acid. Once extracted into solution, the collagen can be salt-precipitated using NaCl and recovered, using standard techniques such as centrifugation or filtration. Details of acid extracted collagen are described, for example, in U.S. 5,106,949.

Collagen dispersions or gels for use in the present invention are generally at a concentration of about 1 to 10 mg/ml, preferably, from about 2 to 6 mg/ml, and most preferably at about 2 to 4 mg/ml and at pH of about 2 to 4. A preferred solvent for the collagen is dilute acetic acid, e.g., about 0.05 to 0.1%. Other conventional solvents for collagen may be used as long as such solvents are compatible.

Additionally, in another embodiment of a prosthesis prepared by the method of the invention, the collagenous layer can include mechanically sheared or chopped collagen fibers. The chopped collagen fibers improve the spring-back performance of the collagenous layer. The chopped fibers can be added to the collagen solution used for formation of the acid-extracted collagen gel. The properties of the construct incorporating the fibers may be varied by variations in the length and diameter of the fiber; variations on the proportion of the fiber used, and partially crosslinking fibers. The length of the fibers can range from 5 cm to 5.0 cm, and will typically be incorporated into the collagen gel at a concentration of 5 to 60.

In another embodiment of a prothesis prepared by the method of the invention, the formation of the inner or outer collagenous layer can incorporate previously formed collagen threads. For example, a helix, or braid of micron diameter collagen thread could be incorporated as part of the formation of the collagen inner layer. The diameter size of the helix or braid of collagen thread can range from 25 to 50 microns, preferably 25 to 40 microns. Thus, the properties of the collagen layer can be varied by the geometry of the thread used for the reinforcement. The functionality of the design is dependent on the geometry of the braid or twist. Many of these will also effect the physical properties (i.e, compliance, radial strength, kink resistance, suture retention). Physical properties of the thread may also be varied by crosslinking.

Some portion or all of the fibers used could be polylactic acid. The physical and degradation properties of the lactic acid fibers themselves can be manipulated by varying the molecular weight, as well as the use of the D or L racemes or a mixture of D/L forms of lactic acid. Other fibers fabricated from degradable polymers could also be used, such as polyglycolic acid, caprolacatone, and polydioxinone.

### Small Diameter Two-Layer Tubular Prosthesis:

### Method of Preparation

To further describe the prosthesis prepared by the method of this invention, the process of making a small diameter two layered tubular prosthesis will be described in detail below. The described two-layered prosthesis has an inner (luminal) surface composed of acid-extracted fibrillar collagen and the outer (abluminal) structural layer composed of mammalian tunica submucosa from the small intestine. Flat prosthesis can be similarly prepared with the described methods by using a flat form instead of a mandrel to produce the prosthesis.

### 1. Preparation of the Structural Layer

The submucosa, or the intestinal collagen layer, from a mammalian source, typically pigs, cows, or sheep, is mechanically cleaned by squeezing the raw material between opposing rollers to remove the muscular layers (tunica muscularis) and the mucosa (tunica mucosa). The tunica submucosa of the small intestine is harder and stiffer than the surrounding tissue, and the rollers squeeze the softer components from the submucosa. As the mechanically cleaned submucosa may have some hidden, visibly nonapparent damage that affects the consistency of the mechanical properties, the submucosa may be chemically cleaned to remove substances other than collagen using, for example, by soaking in buffer solutions at 4°C, without the use of any detergents such as Triton or SDS, or by soaking with NaOH or trypsin, or other known cleaning techniques.

After cleaning, the intestinal collagen layer (ICL) should be sterilized, preferably with the use of dilute peracetic acid solutions as described in U. S. patent application serial number 08/177,618. Other sterilization systems for use with collagen are known in the art and can be used.

The ICL may be tubulated by various alternative means or combinations thereof. The ICL material may be formed into a tube in either the normal or the everted position, but the everted position is preferred. The tube may be made mechanically by suturing, using alternating knot stitches with suitable suture material. The knot stitch is advantageous as it allows the tube to be trimmed and shaped by the surgeon at the time of implantation without unraveling. Other processes to seam the submucosa may include adhesive bonding, such as the use of fibrin-based glues or industrial-type adhesives such as polyurethane, vinyl acetate or polyepoxy. Heat bonding techniques may also be used including heat welding or laser welding of the seam, followed by quenching, to seal the sides of the thus formed tube. Other mechanical means are possible, such as using pop rivets or staples. With these tubulation techniques, the ends of the sides may be either butt ended or overlapped. If the sides are overlapped, the seam may be trimmed once the tube is formed. In addition, these tubulation techniques are typically done on a mandrel so as to determine the desired diameter.

The thus formed structural tube can be kept on a mandrel or other suitable spindle for further processing. To control the biodegradation rates and therefore the rate of prosthesis strength decrease during bioremodeling, the prosthesis is preferably crosslinked, using a suitable crosslinking agent, such as (1-ethyl-3-(3-dimethylaminopropyl) carbodiimide hydrochloride (EDC). Crosslinking the prosthesis also aids in prevent luminal creep, in keeping the tube diameter uniform, and in increasing the burst strength. It is believed that crosslinking the intestinal collagen layer also improves the suture retention strength by improving resistance to crack propagation.

### 2. Deposition of Collagenous Laver(s).

Bovine collagen may be deposited on the internal surface of the submucosa as described in example 5 of U. S. Patent Application 07/505,678. Briefly, the structural intestinal collagen layer is sealed at one end by luer fittings and the collagen dispersion fills the tube. This step may also be accomplished as described in the above-referenced patent application using a hydrostatic pressure head. The inner layer of collagen can also be deposited by flowing collagen into both ends of the tube simultaneously. The tube is then placed into a bath of 20% polyethylene glycol (PEG) in isotonic phosphate buffered saline (PBS), pH about 7. The osmotic gradient between the internal collagen solution and outer PEG solution in combination cause a simultaneous concentration and deposition of the collagen along the lumen of the internal structural layer wall. The tube is then removed from the PEG bath, and a glass rod with a diameter desired diameter of the prosthesis lumen is inserted into the collagen solution. The prosthesis is then allowed to dry. The tube is then rehydrated in PBS. This process allows the collagenous layer to fill slight irregularities in the intestinal structural layer, thus resulting in a prosthesis of uniform thickness. The procedure also facilitates the bonding of the collagen gel to the intestinal collagen layer. A collagenous layer of varying thickness and density can be produced by changing the deposition conditions which can be determined by routine parameter changes. The same procedures can be used to apply the collagen to the outer surface of the submucosa to create a three-layer prosthesis.

### 3. Treatment of the Inner Collagenous Layer.

The prosthesis construct is thrombogenic in small diameter blood vessel replacements. It can only be used in vascular applications in high flow (large diameter) vessels. Therefore, the prosthesis must be rendered non-thrombogenic if to be useful for small diameter blood vessel repair or replacement.

Heparin can be applied to the prosthesis, by a variety of well-known techniques. For illustration, heparin can be applied to the prosthesis in the following three ways. First, benzalkonium heparin (BA-Hep) solution can be applied to the prosthesis by dipping the prosthesis in the solution and then air-drying it. This procedure treats the collagen with an ionically bound BA-Hep complex. Second, EDC can be used to activate the heparin, then to covalently bond the heparin to the collagen fiber. Third, EDC can be used to activate the collagen, then covalently bond protamine to the collagen and then ionically bond heparin to the protamine. Many other heparin coating, bonding, and attachment procedures are well known in the art which could also be used.

Treatment of the inner layer with drugs in addition to heparin may be accomplished. The drugs may include for example, growth factors to promote vascularization and epitheliazation, such as macrophage derived growth factor (MDGF), platelet derived growth factor (PDGF), endothelial cell derived growth factor (ECDGF); antibiotics to fight any potential infection from the surgery implant; or nerve growth factors incorporated into the inner collagenous layer when the prosthesis is used as a conduit for nerve regeneration. The treatment of the abluminal (outer) layer may also be done in a manner similar to that for the luminal (inner layer).

### 4. Cell Ingrowth Facilitation.

If the structural layer is made of ICL which is crosslinked the completed two or three layer prosthesis can be laser drilled to create micron sized pores through the completed prosthesis for aid in cell ingrowth using an excimer laser at either KrF or XeF wavelengths. The pore size can vary from 20 to 100 microns, but is preferably from about 30 to 60 microns and spacing can vary, but about 500 microns on center is preferred.

### 5. Sterilization.

The completed graft is then sterilized. The preferred method is to use peracetic acid as described in U. S. patent application serial number 08/177,618. Sterilization may also be accomplished by subjecting the prosthesis to a gamma radiation treatment (⁶⁰Co) of 10.0 to 25.0 kGy. The radiation dose eliminates all microorganisms without adversely affecting the biomechanical properties of the prosthesis.

### Prosthesis Test Standards

Various tests, analysis and performance parameters have been developed over the years for vascular graft prosthesis and can be used by those of skill in the art to evaluate the prosthesis characteristics. These methods are detailed in Abbott et al., "Evaluation and performance standards for arterial prostheses," Journal of Vascular Surgery, Volume 17, pages 746-756 (1993) and "American National Standard for Vascular Graft Prostheses," American National Standards Institute (1986).

The following examples further describe the materials and methods used in carrying out the invention. The examples are not intended to limit the invention in any manner.

### EXAMPLES

### Example 1

### Two and Three Layer Tubular Prosthesis

The small intestine of a pig was harvested and mechanically stripped so that the tunica submucosa is delaminated from the tunica muscularis and the luminal portion of the tunica mucosa of the section of small intestine. (The machine was a striper, crusher machine for the mechanical removal of the mucosa and muscularis layers from the submucosal layers using a combination of mechanical action (crushing) and washing using hot water.) This was accomplished by running the intact intestine through a series of rollers that strip away successive layers. The intestinal layer was machine cleaned so that the submucosa layer solely remains. The submucosa was decontaminated or sterilized with 0.1% peracetic acid for 18 hours at 4°C and then washed after the peracetic acid treatment.

This machine cleaned intestinal collagen layer (ICL) was mounted and stretched on a frame so that it was under slight tension both radially and longitudinally. Coarse running basting stitches (6-0 Novafil®) were applied to form a small diameter tube, with the submucosa in the everted position. The stretched ICL tissue was cut in half to overlap and form flaps. A fine seam through both layers of ICL was formed using 6-0 Novafil® suture with an alternating knot stitch so that a final internal diameter of 4.5 mm was obtained. The flaps were then removed. The small-diameter ICL tube, used as the structural layer, was placed onto a 4.5 mm glass rod and crosslinked with 100 mmol EDC (Pierce) for 18 hours at room temperature.

The machine cleaned intestinal collagen layer (ICL) in a fully hydrated condition was mounted and wrapped on a mandrel so that the ends overlapped. The ICL wrapped mandrel was heated to 62°C plus or minus 10°C for 15 minutes in a moist atmosphere, followed by quenching at 4°C in iced aqueous solution for 5 minutes. The tubulated ICL was then crosslinked with EDC for 6 to 18 hours, rinsed, and removed from the mandrel.

Polycarbonate barbs (luer lock fittings that are funnel shaped on one end) were placed tightly in either end of the tube and then the tube was placed horizontally in a deposition fixture. A 15 ml reservoir of 2.5 mg/ml acid-extracted fibrillar collagen, termed "dense fibrillar collagen" ("DFC") (U. S. patent application, serial no. 07/772,579) with a hydrostatic pressure head of 150 mmHg (for 5 feet) was attached via the barbs. (The pressure will depend on the height of the collagen reservoir). The collagen was allowed to fill the lumen of the ICL tube and was then placed into a stirring bath of 20% MW 8000 polyethylene glycol (Sigma Chemical Co.) for 16 hours at 4°C. The apparatus was then dismantled. To fix the luminal diameter, a 4 mm diameter glass rod was placed into the collagen-filled ICL tube. The prosthesis was then allowed to dry for 18 hours at 4° C.

A layer of acid extracted fibrillar collagen was deposited onto a 4.0 mm diameter porous ceramic mandrel as described in Example 4, U. S. Patent Application 07/505,678, for 6 hours and dehydrated at 4°C.

The ICL tube, as described above, was placed over the dried collagen and a second layer of dense fibrillar collagen (DFC), as described above, was applied for 18 hours to the outside (abluminal) of the ICL.

Pores were drilled in the ICL/DFC or the DFC/ICL/DFC using an excimer laser at either KrF or XeF wavelengths. The pore size was about 50 microns and spacing was 500 microns on center.

The construct was rehydrated in 4°C 1M PBS for 6 hours. The prosthesis was treated with application of benzalkonium heparin in isopropranol. Sterilization was accomplished with 0.1% peracetic acid for 18 hours at 4°C.

The prosthesis was packaged and sterilized with 10.0 to 25.0 kGy of gamma radiation (⁶⁰Co). (The prosthesis can also be shipped dry and rehydrated in saline, prior to implantation.)

### Example 2

### Remodeling of the Collagen Graft: Long Term Implant Histology

Three-layer prosthesis were implanted in the infra-renal aorta of rabbits using standard surgical techniques. Proline, 7-0, was used to construct end to end anatomoses to the adjacent arteries. The grafts were 1.5 cm in length and 3 mm in diameter. No anti-platelet medications were administered post-operatively.

Following pressure perfusion with McDowells-Trump fixative, the grafts were explanted, and submitted for light and electron microscopy. Specimens from 30, 60, 90, 120, and 180 day implants were available. Materials were examined with H/E, VonGieson elastica, Masson's Trichrome, g-Actin, Factor VIII, and Ram-11 (macrophage) stains, and polarized microscopy. Qualitative morphometric comparisons were made to stained non-implanted retention samples.

Histological evaluations demonstrated that the graft was readily invaded by host cells. The luminal collagen was resorbed and remodeled with the production of new collagen by host myofibroblasts. The ICL was readily invaded, repopulated by host cells, and remodeled. Endothelial cells were demonstrated on the luminal surface of the prosthesis.

At 30 days, large numbers of mononuclear inflammatory cells were seen on both the luminal and abluminal surfaces of the collagen. Modest numbers of Ram-11 positively staining macrophages, were observed. At the surface of the cast collagen, there was cell mediated collagen resporption and remodeling. There was minimal loss of the collagen bulk at this time.

At 60 days, the cellular response was more myofibroblastic than inflammatory. Significant amounts of new collagen as well as small amounts of elastin were readily identified. About 50 percent of the cast collagen had been remodeled. Endothelial cells as identified by SEM appearance, TEM (Weibel-Palade bodies) and positive Factor VIII staining, covered the surface of the remodeling construct.

At 90 days, the matrix surrounding the myofibroblasts (as identified with g-actin) stained prominently for collagen. The cytoplasm of cells themselves had reduced amounts of cytoplasm as compared to previous timepoints.

At 120 days, the stroma demonstrated well organized predominantly radially and longitudinally oriented myofibroblasts and host produced collagen. Significant amounts of elastin could be identified. Greater than 90 percent of the implanted collagen had been remodeled. No Ram-11 staining macrophages were identified.

At 180 days, cells and the matrix of the neo-artery were quite mature. The cells were small with minimal cytoplasm. The collagen was dense and distinctly radially and longitudinally oriented.

There was no histological evidence of an immune response to either the luminal collagen layer or the abluminal ICL layer. No grafts became dilated or aneurysmal.

### Example 3

### Comparison of Three-Layer Prosthesis and e-PTFE

Both two-layer and three-layer small diameter prosthesis were implanted and evaluated over time for patency and remodeling.

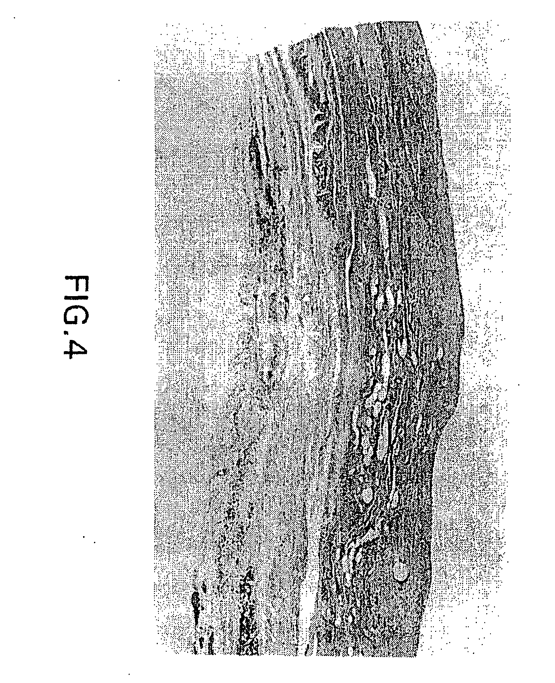

Figures 4-9 show the results of a comparison of three-layer prothesis with a similarly configured contra-lateral reference material, e-PTFE, in a canine femoral artery study. The grafts were implanted in canines as femoral interposition prosthesis. Grafts were explanted from 30 to 256 days.

Histological evaluation of the three-layer collagen graft demonstrated cellular ingrowth into the graft at 30 days, with more than 90 percent of the graft collagen remodeled by 90 days; and a mature 'neo-artery' at 180 days. Host tissue bridged the anastomosis by 60 days with the anastomosis only demarcated by the non-resorbable sutures. The predominant cell type in the neo-artery was a positive g-Actin staining smooth muscle like cell. The surface of the remodeled graft was lined by endothelial cells as demonstrated by SEM, TEM and Factor VIII staining.

In contrast, at times to 256 days, no ingrowth into the e-PTFE artery was observed either across the anastomosis or along the body of the graft. Only a thin smooth muscle cell hyperplastic response was demonstrated extending from the adjacent artery a short distance on the graft's luminal surface. The graft was encapsulated by mature fibrous tissue with no evidence of cellular or tissue extension into the graft.

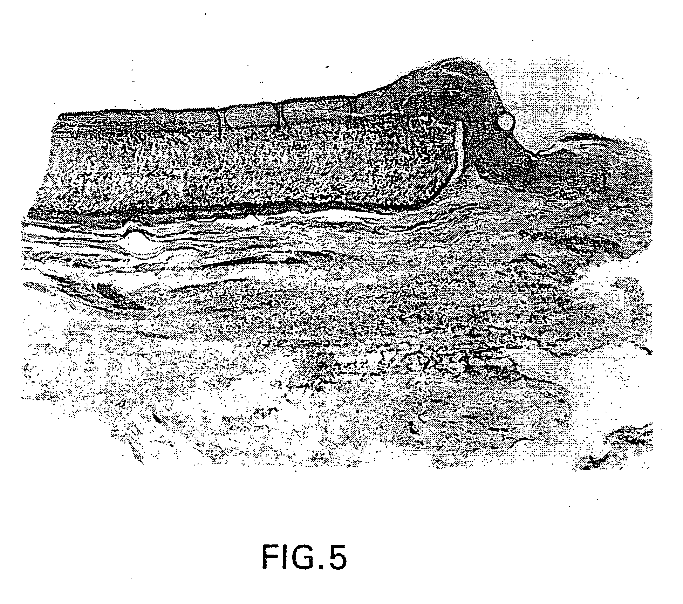

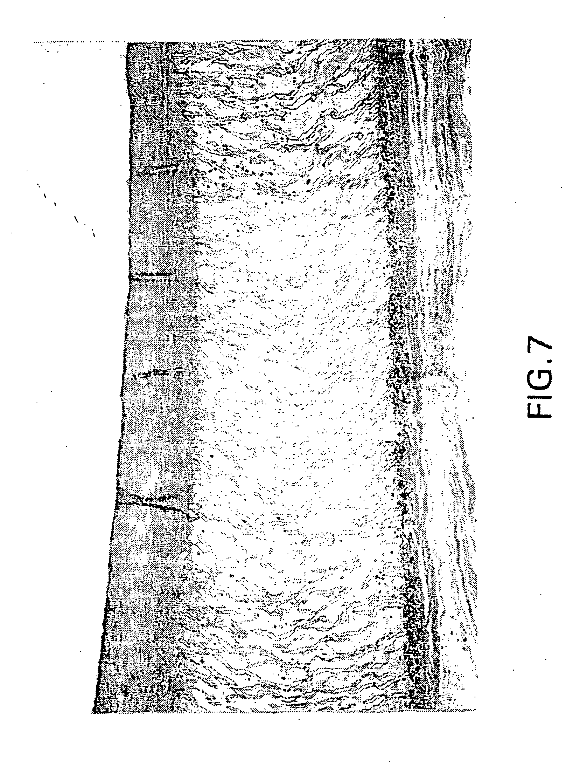

Figure 4 is a Masson's trichrome stain (10x) of the proximal anastomosis of the three-layer prosthesis at 256 days compared with Figure 5 of an e-PTFE graft. Figure 6 is also a Masson's trichrome stain at 25x of the proximal anastomosis of a three-layer prosthesis at 256 days compared with Figure 7 of an e-PTFE graft.

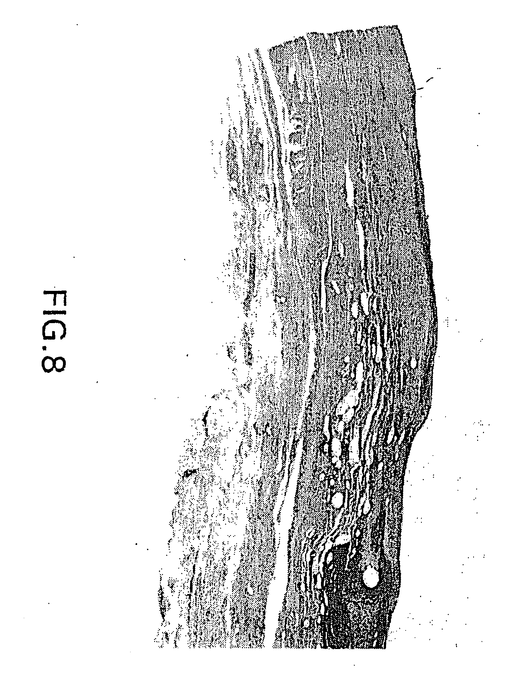

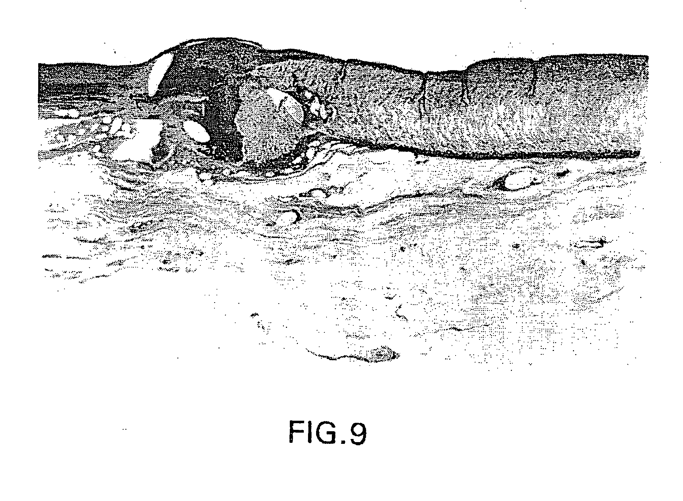

Figure 8 is a Verhoeff's elastic stain (10x) of the proximal anastomosis of a three-layer prosthesis implanted as a canine femoral interposition prosthesis at 256 days compared with Figure 9 of an e-PTFE graft.

Although the foregoing invention has been described in detail by way of illustration and example for purposes of clarity of understanding, it will be obvious to one skilled in the art that changes and modifications may be practiced within the scope of the invention, as limited only by the scope of the appended claims.

## Claims

1. A method for preparing a bioremodelable prosthesis having at least two layers, comprising:
a) forming a first structural layer that is pliable, semi-permeable, and suturable; and
b) forming a second layer to act as a smooth flow surface comprising deposition of acid extracted fibrillar or non-fibrillar collagen onto a surface of said structural layer of step a).

2. A method as claimed in claim 1, wherein the structural layer is made from bioremodelable collagen or collagenous materials; or biodegradable polymeric materials, such as polylactic or polyglycolic acid, or combinations thereof.

3. A method as claimed in claim 2, wherein collagenous material from collagenous parts of tissue from the mammalian body is used to make the structural layer.

4. A method as claimed in claim 3, wherein the structural layer is made from the tunica submucosa layer of the small intestine.

5. A method as claimed in claim 3 or 4, wherein the collagenous material is cross-linked with a cross-linking agent.

6. A method as claimed in claim 5, wherein the cross-linking agent is 1-ethyl-3-(3-dimethylaminopropyl) carbodiimide hydrochloride (EDC).

7. A method as claimed in any one of claims 4 to 6, wherein the tunica submucosa of the small intestine is mechanically cleaned to remove the tunica muscularis and the tunica mucosa.

8. A method as claimed in claim 7, wherein said mechanically cleaned tunica submucosa is chemically cleaned.

9. A method as claimed in any one of claims 4 to 8, wherein the tunica submucosa is sterilised.

10. A method as claimed in any one of the preceding claims, wherein the acid extracted fibrillar or non fibrillar collagen is prepared by acid extraction of a collagen source to result in a fibril collagen dispersion or gel.

11. A method as claimed in claim 10, wherein the collagen is acid extracted from the collagen source using a weak acid, such as acetic, citric or formic acid.

12. A method as claimed in claim 10 or 11, wherein after acid extraction the collagen is salt-precipitated and recovered.

13. A method as claimed in any one of claims 10 to 12, wherein the collagen dispersion is at a concentration of about 1 to 10 mg/ml.

14. A method as claimed in any one of claims 10 to 13, wherein the collagenous layer includes mechanically sheared or chopped collagen fibres.

15. A method as claimed in any one of claims 10 to 13, wherein collagen threads are incorporated in the collagen layer.

16. A method as claimed in claim 15, wherein a helix or braid of collagen thread is incorporated.

17. A method as claimed in claim 16, wherein the diameter size of the helix or braided thread is from 25 to 50 microns.

18. A method as claimed in any one of the preceding claims, wherein in step a) the first structural layer is formed into a tube.

19. A method as claimed in claim 18, wherein the first layer is formed into a tube by suturing, adhesive bonding, heat bonding, or by mechanical means.

20. A method as claimed in claim 18 or 19, wherein in step b), the second layer is deposited on the internal surface of the tube of the first layer.

21. A method as claimed in claim 20, wherein the first layer is sealed at one end and the collagen dispersion fills the tube.

22. A method as claimed in claim 20, wherein in step b), the second layer is deposited on the internal surface of the tube of the first layer by flowing collagen dispersion into both ends of the tube of the first layer simultaneously.

23. A method as claimed in any one of claims 20 to 22, wherein the tube is then placed in 20% polyethylene glycol (PEG) in isotonic phosphate buffered saline (PBS) at a pH of about 7.

24. A method as claimed in claim 23, wherein a rod with a diameter desired for the lumen of the prothesis is inserted into the collagen dispersion solution.

25. A method as claimed in any one of the preceding claims, wherein the second layer is treated with an anti-thrombic drug.

26. A method as claimed in any one of the preceding claims, wherein the method further comprises depositing a further layer of acid extracted fibrillar or non fibrillar collagen onto the external surface of the first structural layer, to form a third layer.

## Patentansprüche

1. Ein Verfahren zur Herstellung einer bioremodellierbaren Prothese, welche zumindest zwei Schichten aufweist, umfassend:
a) Formen einer ersten Strukturschicht, die biegsam, halbdurchlässig und nähbar ist; und
b) Formen einer zweiten Schicht, die als glatte Strömungsfläche agieren soll,
umfassend die Ablagerung eines mit Säure extrahierten fibrillären oder nicht-fibrillären Kollagens auf eine Fläche der Strukturschicht von Schritt a).

2. Das Verfahren gemäß Anspruch 1, wobei die Strukturschicht aus bioremodellierbaren Kollagen oder kollagenen Materialien; oder biologisch abbaubaren polymerischen Materialien, wie Polymilch- oder Polyglycolsäure, oder Kombinationen davon, hergestellt wird.

3. Das Verfahren nach Anspruch 2, wobei das kollagene Material aus kollagenen Gewebeteilen eines Körpers eines Säugetiers zur Herstellung der Strukturschicht verwendet wird.

4. Das Verfahren nach Anspruch 3, wobei die Strukturschicht aus der Tunica-Submucosa-Schicht des Dünndarms hergestellt ist.

5. Das Verfahren nach Anspruch 3 oder 4, wobei das kollagene Material mit einem Vernetzungsmittel vernetzt ist.

6. Das Verfahren nach Anspruch 5, wobei das Vernetzungsmittel 1-Ethyl-3-(3-dimethylaminopropyl)carbodiimid Hydrochlorid (EDC) ist.

7. Das Verfahren nach einem der Ansprüche 4 bis 6, wobei die Tunica submucosa des Dünndarms mechanisch gereinigt wird, um die Tunica muscularis und die Tunica mucosa zu entfernen.

8. Das Verfahren nach Anspruch 7, wobei die mechanisch gereinigte Tunica submucosa chemisch gereinigt wird.

9. Das Verfahren nach einem der Ansprüche 4 bis 8, wobei die Tunica submucosa sterilisiert wird.

10. Das Verfahren nach einem der vorangehenden Ansprüche, wobei das mit Säure extrahierte fibrilläre oder nicht-fibrilläre Kollagen durch Säureextraktion einer Kollagenquelle hergestellt wird, um eine/ein fibrilläre/s Kollagendispersion oder -gel zu erhalten.

11. Das Verfahren nach Anspruch 10, wobei das Kollagen ein aus der Kollagenquelle säureextrahiertes Kollagen ist, unter Verwendung einer schwachen Säure, wie Essig-, Zitrus- oder Ameisensäure.

12. Das Verfahren nach Anspruch 10 oder 11, wobei nach der Säureextraktion das Kollagen mit Salz präzipitiert und gewonnen wird.

13. Das Verfahren nach einem der Ansprüche 10 bis 12, wobei die Kollagendispersion bei einer Konzentration von ca. 1 bis 10 mg/ml liegt.

14. Das Verfahren nach einem der Ansprüche 10 bis 13, wobei die kollagene Schicht mechanisch gescherte oder gehackte Kollagenfasern umfasst.

15. Das Verfahren nach einem der Ansprüche 10 bis 13, wobei die Kollagenfäden in die Kollagenschicht eingearbeitet sind.

16. Das Verfahren nach Anspruch 15, wobei eine Helix oder Flechte eines Kollagenfadens eingearbeitet ist.

17. Das Verfahren nach Anspruch 16, wobei die Durchmessergröße des helixförmigen oder geflochtenen Fadens zwischen 25 und 50 Mikrometer liegt.

18. Das Verfahren nach einem der vorangehenden Ansprüche, wobei die erste Strukturschicht in Schritt a) zu einem Rohr geformt wird.

19. Das Verfahren nach Anspruch 18, wobei die erste Schicht durch Nähen, Klebeverbindung, Hitzeverbindung oder durch mechanische Mittel zu einem Rohr geformt wird.

20. Das Verfahren nach Anspruch 18 oder 19, wobei die zweite Schicht in Schritt b) auf der Innenfläche des Rohrs der ersten Schicht abgelagert wird.

21. Das Verfahren nach Anspruch 20, wobei die erste Schicht an einem Ende versiegelt ist und die Kollagendispersion das Rohr ausfüllt.

22. Das Verfahren nach Anspruch 20, wobei die zweite Schicht in Schritt b) auf der Innenfläche des Rohrs ersten Schicht **dadurch** abgelagert wird, dass die Kollagendispersion gleichzeitig in beide Enden des Rohrs der ersten Schicht fließt.

23. Das Verfahren nach einem der Ansprüche 20 bis 22, wobei das Rohr dann in 20% Polyethylenglycol (PEG) in einer Phosphat-gepufferten Kochsalzlösung (PBS) bei einem pH-Wert von ca. 7 platziert wird.

24. Das Verfahren nach Anspruch 23, wobei eine Stange mit einem Durchmesser, der für das Lumen der Prothese erwünscht ist, in die Lösung der Kollagendispersion eingeführt wird.

25. Das Verfahren nach einem der vorangehenden Ansprüche, wobei die zweite Schicht mit einem anti-thrombischen Medikament behandelt wird.

26. Das Verfahren nach einem der vorangehenden Ansprüche, wobei das Verfahren zusätzlich die Ablagerung einer weiteren Schicht eines mit Säure extrahierten fibrillären oder nicht-fibrillären Kollagens auf die Außenfläche der ersten Strukturschicht zur Bildung einer dritten Schicht umfasst.

## Revendications

1. Procédé pour préparer une prothèse bioremodelable ayant au moins deux couches, comprenant :
a) la formation d'une première couche structurelle pliable, semi-perméable et pouvant être suturée; et
b) la formation d'une deuxième couche destinée à jouer le rôle de surface d'écoulement lisse, comprenant le dépôt de collagène fibrillaire ou non fibrillaire obtenu par extraction à l'acide, sur une surface de ladite couche structurelle de l'étape a).

2. Procédé selon la revendication 1, dans lequel la couche structurelle est produite à partir de collagène bioremodelable ou de matériaux collagéniques; ou de matériaux polymères biodégradables, tels que l'acide polylactique ou l'acide polyglycolique ou leurs combinaisons.

3. Procédé selon la revendication 2, dans lequel le matériau collagénique, provenant de parties collagéniques d'un tissu d'un corps de mammifère, est utilisé pour fabriquer la couche structurelle.

4. Procédé selon la revendication 3, dans lequel la couche structurelle est produite à partir d'une couche de sous-muqueuse de l'intestin grêle.

5. Procédé selon la revendication 3 ou 4, dans lequel le matériau collagénique est réticulé avec un agent de réticulation.

6. Procédé selon la revendication 5, dans lequel l'agent de réticulation est le chlorhydrate de 1-éthyl-3-(3-diméthylaminopropyl)carbodiimide (EDC).

7. Procédé selon l'une quelconque des revendications 4 à 6, dans lequel la sous-muqueuse de l'intestin grêle est nettoyée mécaniquement pour éliminer la tunique musculaire et la muqueuse.

8. Procédé selon la revendication 7, dans lequel ladite sous-muqueuse nettoyée mécaniquement est nettoyée chimiquement.

9. Procédé selon l'une quelconque des revendications 4 à 8, dans lequel la sous-muqueuse est stérilisée.

10. Procédé selon l'une quelconque des revendications précédentes, dans lequel on prépare le collagène fibrillaire ou non fibrillaire par extraction à l'acide d'une source de collagène, dans le but d'obtenir une dispersion ou un gel de collagène fibrillaire.

11. Procédé selon la revendication 10, dans lequel le collagène est extrait à l'acide à partir de la source de collagène en utilisant un acide faible, tel que l'acide acétique, l'acide citrique ou l'acide formique.

12. Procédé selon la revendication 10 ou 11, dans lequel, après l'étape d'extraction à l'acide, on provoque la précipitation du collagène en milieu salin et on le récupère.

13. Procédé selon l'une quelconque des revendications 10 à 12, dans lequel la dispersion de collagène présente une concentration d'environ 1 à 10 mg/ml.

14. Procédé selon l'une quelconque des revendications 10 à 13, dans lequel la couche collagénique comprend des fibres de collagène qui ont été cisaillées ou hachées mécaniquement.

15. Procédé selon l'une quelconque des revendications 10 à 13, dans lequel on incorpore des filaments de collagène à la couche de collagène.

16. Procédé selon la revendication 15, dans lequel on incorpore une structure hélicoïdale ou tressée de filaments de collagène.

17. Procédé selon la revendication 16, dans lequel le diamètre du filament de structure hélicoïdale ou tressée est de 25 à 50 micromètres.

18. Procédé selon l'une quelconque des revendications précédentes, dans lequel, à l'étape a), la première couche structurelle est formée dans un tube.

19. Procédé selon la revendication 18, dans lequel la première couche est formée dans un tube par suture, par fixation adhésive, par fixation thermique ou par des moyens mécaniques.

20. Procédé selon la revendication 18 ou 19, dans lequel, à l'étape b), la deuxième couche est déposée sur la surface interne du tube de la première couche.

21. Procédé selon la revendication 20, dans lequel la première couche est scellée à une extrémité et la dispersion de collagène remplit le tube.

22. Procédé selon la revendication 20, dans lequel, à l'étape b), la deuxième couche est déposée sur la surface interne du tube de la première couche en faisant couler la dispersion de collagène simultanément dans les deux extrémités du tube de la première couche.

23. Procédé selon l'une quelconque des revendications 20 à 22, dans lequel le tube est ensuite placé dans du polyéthylène glycol (PEG) à 20% dans une solution isotonique tamponnée aux sels de phosphate (PBS) au pH d'environ 7.

24. Procédé selon la revendication 23, dans lequel une tige de diamètre souhaité pour la lumière de la prothèse est inséré dans la solution de dispersion de collagène.

25. Procédé selon l'une quelconque des revendications précédentes, dans lequel la deuxième couche est traitée avec un médicament antithrombique.

26. Procédé selon l'une quelconque des revendications précédentes, dans lequel le procédé comprend en outre le dépôt d'une autre couche de collagène fibrillaire ou non fibrillaire, extrait à l'acide, sur la surface externe de la première couche structurelle, pour former une troisième couche.
